# EUROPEAN PATENT SPECIFICATION

(11) **EP 2 436 693 B1**
(45) Date of publication and mention of the grant of the patent: **20.01.2016**
(21) Application number: 11176165.6
(22) Date of filing: 01.08.2011
(51) Int. Cl.: C07K 14/47, C12N 15/74

(54) **Synthetic genes encoding peptide fragments of natural myelin proteins for induction of oral tolerance, DNA fragment comprising these genes, means of obtaining these peptides in a microbial (bacterial) system and their medical application**
Synthetische Gene, die Peptidfragmente von natürlichen Myelinproteinen kodieren, für die Induktion oraler Toleranz, DNA-Fragment enthaltend diese Gene, Mittel zur Gewinnung dieser Peptide in einem microbiellen (bakteriellen) System und ihre medizinische Anwendung.
Gènes synthétiques, codant pour des fragments peptidiques de protéines de myélines naturelles, pour une induction de la tolérance orale, fragments d'ADN comprenant ces gènes, moyens d'obtention de ces peptides dans un système microbian et leurs applications thérapeutiques.

(30) Priority: 02.08.2010 PL 39203710
(43) Date of publication of application: 04.04.2012
(73) Proprietor: Instytut Biochemii i Biofizyki PAN, 02-106 Warszawa (PL)
(72) Inventor: Szczepankowska, M. Agnieszka, 00-710 Warszawa (PL); Bardowski, Jacek, 01-179 Warszawa (PL); Aleksandrzak-Piekarczyk, Tamara, 01-318 Warszawa (PL); Kasarello, Kaja, 02-793 Warszawa (PL); Lipkowski, Andrzej W., 02-786 Warszawa (PL); Kwiatkowska- Patzer, Barbara, 05-500 Piaseczno (PL)
(74) Representative: Grzelak, Anna

(56) References cited:
- WO-A2-01/31037
- WO-A2-2006/123230
- MAASSEN C B M ET AL: "Reduced experimental autoimmune encephalomyelitis after intranasal and oral administration of recombinant lactobacilli expressing myelin antigens", VACCINE, ELSEVIER LTD, GB, vol. 21, no. 32, 1 December 2003 (2003-12-01), pages 4685-4693, XP004469685, ISSN: 0264-410X, DOI: 10.1016/S0264-410X(03)00522-X
- MILLER A ET AL: "EPITOPES OF MYELIN BASIC PROTEIN THAT TRIGGER TGF-BETA RELEASE AFTER ORAL TOLERIZATION ARE DISTINCT FROM ENCEPHALITOGENIC EPITOPES AND MEDIATE EPITOPE-DRIVEN BYSTANDER SUPPRESSION", JOURNAL OF IMMUNOLOGY, AMERICAN ASSOCIATION OF IMMUNOLOGISTS, US, vol. 151, no. 12, 15 December 1993 (1993-12-15), pages 7307-7315, XP002919991, ISSN: 0022-1767
- Kwiatkowska-Patzer Barbara (Abstract #68): "Spinal cord peptide epitopes ameliorate immunological reaction in experimental allergic encephalomyelitis" In: "Book of Abstracts: The Seventh Multidisciplinary Converence on Drug Research", November 2011 (2011-11), Pielaszek Research, XP002667932, ISBN: 83-89585-29-4 page 63, * publicly disclosed at poster session on 11.05.2010 * * the whole document *
- KWIATKOWSKA-PATZER BARBARA ET AL: "Spinal cord hydrolysate ameliorate immunological reaction in experimental allergic encephalomyelitis.", ACTA NEUROBIOLOGIAE EXPERIMENTALIS 2009 LNKD- PUBMED:19325643, vol. 69, no. 1, 2009, pages 73-78, XP002667933, ISSN: 0065-1400

## Description

### Technical Field

The subject of the invention is a synthetic gene encoding low molecular weight myelin protein, derivative of protein fragments of mammalian spinal cord, for use in the specific induction of oral tolerance.

### Background Art

Multiple sclerosis is a destructive disease of the central nervous system that affects mainly young people, between 20 and 40 years of age. Incidence data indicate that around 60 000 people suffer from this disease in Poland. It is an autoimmune disease, for which, as yet, there is no effective treatment. Immunomodulatory drugs, such as interferon and Copaxone, only change the course of the disease, reducing the number of attacks, but after 6 years of treatment, the disability of people with multiple sclerosis is the same as without treatment. Moreover, treatment of this disease is quite expensive, and reimbursement of medical expenses - infrequent. Hence the need for intensive research on new effective and economically profitable methods of treating this chronic immune disease.

Autoimmune diseases are based on a pathological recognition by the immune system of the body's own proteins as foreign antigens, which, in consequence, leads to induction of mechanism eliminating these antigens. In multiple sclerosis, fragments of myelin proteins of the central nervous system constitute antigens pathologically recognized by antibodies.

The mechanism of food tolerance, in which the immune system is specifically desensitized in respect to food nutrients has been made known. It was proposed to use this mechanism for tolerance induction for protein epitopes in autoimmune diseases, such as rheumatism and multiple sclerosis. The Authors' own studies demonstrate that hydrolysates of the spinal cord of farm animals, containing low molecular weight myelin peptide fragments, exhibit an activity that modulates the course of experimental allergic encephalomyelitis (EAE) in an animal model of multiple sclerosis (MS) (1).

Undertaking studies aimed at developing a method of producing myelin peptides in lactic acid bacteria was associated with a number of premises: (i) the important status of multiple sclerosis among diseases occurring in Poland; (ii) low efficiency and high costs of medical treatment of this disease; (iii) demonstrated effectiveness of peptides in therapy of this disease unit; (iv) the documented role of the gut (gastrointestinal tract) in the immune system of the organism; (v) previous positive results of using lactic acid bacteria as producers of immunomodulatory factors (cytokines and antigens), including oral vaccines; (vi) data indicating that bacteria intensify the effect of oral tolerance (2).

### CURRENT STATE OF KNOWLEDGE

The recognized targets in the attack on the immune system in multiple sclerosis and in the experimental allergic encephalomyelitis (EAE) animal model are myelin proteins. Main proteins components of myelin are three proteins:
(i) myelin basic protein (MBP), which human amino acid sequence is represented by formula **13** (3)
(ii) proteolipid protein (PLP), which human amino acid sequence is represented by formula **14** (4)
(iii) myelin-oligodendrocyte glycoprotein (MOG), which human amino acid sequence is represented by formula **15** (5)

Studies performed during the last decade deliver evidence that peptide fragments, which are products of partial hydrolysis of dietary proteins, have also the ability to penetrate the gut barrier. Some short peptides penetrate this barrier more easily than single amino acids. To differentiate food peptides penetrating the gut-blood barrier from invasive bacterial and viral proteins, organisms have developed a specific system of food antigen presentation, in result of which the specific immunogenicity for certain peptide sequences (and also other, non-peptide nutrient components) is reduced. This phenomenon has been termed oral tolerance.

The present invention is based on own preliminary research. Application of a spinal cord hydrolysate preparation in a arbitrarily selected dose in rats with EAE led to reduction of inflammatory symptoms both when applied before and after EAE induction (6).

Exploitation of lactic acid bacteria as biological factories producing peptide fragments of natural myelin proteins is of great scientific and application interest and presents many beneficial medical and pharmacological aspects.

Lactic acid bacteria (LAB) constitute a large and taxonomically diverse group of Gram-positive bacteria. An indisputable advantage of these bacteria is their granted GRAS status (Generally Regarded As Safe), i.e. non-pathogenic and safe bacteria for humans and animals. Lactic acid bacteria are a significant object of biotechnological uses as well as basic and applicational research. Biotechnological use of lactic acid bacteria finds application in various industrial branches (7) - food (e.g. production of fermented milk products, food and feeds as well as feed additives), chemical (e.g. production of polymers and enzymes) and pharmaceutical (e.g. probiotic preparation, such as Lakcid or Lactovaginal). In recent years great hopes have been connected with the use of these bacteria in production of functional food, probiotics as well as drugs in the form of oral vaccines (7, 8, 9). In respect to the latter, high expectations are associated with exploiting lactic acid bacteria as vaccine vectors (10-12). Also, over the last few years an increase in research was observed aimed at applying lactic acid bacteria to produce different proteins of therapeutic or prophylactic significance. Lactic acid bacteria are attempted to be used in prophylaxis and treatment of a syndrome known as the inflammatory bowel disease (IBD), which includes also the Crohn's disease and ulcerative colitis.

The aim of the present inventors was the development of a biological system, based on lactic acid bacteria, for production of peptide fragment of natural myelin proteins, to create a preparation for induction of oral tolerance.

Peptide fragment of natural myelin protein for use in the invention was selected based on own studies, which indicated that patients with multiple sclerosis are identified with the HLA-DR2 antigen that recognizes fragment 84-103 of the MBP protein (13). Antibodies against other fragments of the three main proteins are also identified (14, 15) and during progression of the disease the number of recognized antigens deriving from the three basic myelin proteins increases. The above observations are most probably connected with the availability of these proteins as well as their fragments in patients. Immunogenicity of the selected peptide fragments of natural myelin proteins was confirmed also on EAE animal models (16, 17, 18).

Unexpectedly, during implementation of the invention it occurred that it is possible to:
1. . generate by PCR method synthetic genes encoding the selected peptide fragment of natural myelin protein;
2. clone, in cells of selected strains of Gram-positive lactic acid bacteria from *Lactococcus* genus, the obtained recombinant genes in adequate cloning vectors;
3. express in selected lactic acid bacteria (*Lactococcus, Bifidobacterium, Lactobacillus*) genes encoding peptide fragments of natural myelin proteins;
4. clone genes encoding peptide fragments of natural myelin proteins in cells of Gram-negative bacteria *(Escherichia coli);*
optimize the biosynthesis of myelin peptides in lactic acid bacteria

### Summary of invention

Synthetic gene for use in the induction of oral tolerance, wherein the gene contains the MBP21-40 nucleotide fragment ATGGATCATGCTCGTCATGGTTTTTTACCACGTCATCGTGATACTGGTATTTTAG ATTCA (formula 1), encoding a peptide consisting of amino acid sequence MDHARHGFLPRHRDTGILDS (formula 1a), said peptide being a fragment of a natural myelin protein..

The method of generating the gene encoding peptide fragment of natural myelin protein, according to the invention, is based on synthesizing by PCR method of synthetic gene encoding peptide fragment of nautral myelin proteins using 2 complementary primers ("LONG") presented in Table 1 specific for the particular peptide, which nucleotide sequence was designed in such a way so they would correspond to the codons preferably occurring in *Lactococcus lactis* bacteria. Additionally, nucleotide sequences of "FLONG" primers (forward LONG) for peptides MBP85-97, PLP139-151 and PLP178-191 are disclosed, that were modified by introducing a sequence corresponding to the translation START codon (ATG), just before the sequence encoding the first amino acid of the original peptide sequence. At the same time the 5' ends of primers "FLONG_peptide" contain an additional, typical for *Lactococcus lactis* bacteria, RBS (ribosome-binding site) sequence recognized by the translation machinery and a 'spacer' sequence (linking sequence) localized between the RBS region and the translation START codon. Primers "LONG" are used as templates for synthesizing by PCR technique the synthetic genes using short primers ("SHORT") homologous to the 5' ends of primers "FLONG_peptide" and "RLONG_peptide", where the 5' ends of primers "SHORT" carry additional sequences recognized by specific restriction enzymes, preferably Sall (for forward primer SHORT) and Pstl (for reverse primer SHORT), while the 5' ends of prmiers "revSHORT_peptide" (reverse SHORT) have additionally a twice repeated sequence corresponding to the translation STOP codon (TAA) (Table 2).

Disclosed are also DNA fragments obtained through the above-mentioned PCR reaction using primers "LONG" and "SHORT", which nucleotide sequences, , comprise the sequence of synthetic genes of selected peptides of natural myelin proteins (underlined sequences), according to the invention, represented by formula 6, formula 7, formula 8, formula 9 and formula 10.

The method of cloning the generated DNA fragment comprising synthetic gene, according to the invention, is based on subjecting it to digestion by restriction enzymes, favorably Pstl and Sall, and then ligating it with a plasmid vector that replicates in lactic acid bacteria cells, favorably in *Lactococcus,* in particular with the pIL253 vector, digested beforehand with the same restriction enzymes, to obtain recombinant plasmids, in which the orientation of the cloned PCR products (inserts) is in accordance with the direction of transcription directed from the internal promoter present on the vector, in order to introduce the recombinant DNA by electroporation method into cells of bacterial strains, which are cultured in a known manner. Cells containing the new gene are isolated from the culture population, favorably by analyzing the obtained transformants by PCR technique for the presence of inserts which length corresponds to the length of DNA fragments comprising the expected synthetic genes. Nucleotide sequences of the cloned genes are examined for conformity with the nucleotide sequence of the synthetic gene by DNA sequencig technique.

In the cloning method, it is favorable to use as Gram-positive lactic acid bacterial strains into which recombinant DNA is introduced, *Lactococcusstrains,* e.g. *Lactococcus lactis* IL1403, *Lactococcus lactis* IBB477 and *Lactococcus lactis* IBB360, differing in their viability in the mammalian gut as well as other genera of lactic acid bacteria, preferably *Lactobacillus* and *Bifidobacterium.*

The method of producing selected peptides in a bacterial system, is based on expression of synthetic genes of selected peptides in cells of lactic acid bacteria strains, in particular *Lactococcus,* by culturing them in a known medium specific for lactic acid bacteria, preferably on M17 medium or defined CDM medium (*chemically defined medium),* supplemented with sugar, favorably glucose or cellobiose, at a concentration no less than 0.5% or on milk, whey or other suitable medium, at a temperature ranging between 20°C-30°C, preferably at 30°C.

The method of cloning the synthetic genes of selected myelin peptides in Gram-negative bacteria, favorably *Escherichia coli* species, is based on ligation of the synthetic gene amplified using specific primers 'SHORT' (Table 2) with the vector, preferably with a commercially available pGEMT-Easy plasmid or other vector replicating in Gram-negative bacterial cells, favorably from *Escherichia coli* species, to obtain recombinant plasmids. Recombinant DNA is introduced by a known method, e.g. electroporation, to cells of bacterial strains, which are then cultured in a known manner. Cells containing the new gene are isolated from the cultured population, favorably by analyzing the obtained transformants by PCR technique for the presence of inserts which length corresponds to the length of DNA fragments comprising the expected genes. Nucleotide sequences of the cloned DNA fragments are examined for conformity with the nucleotide sequences of the synthetic genes by DNA sequencing technique.

In the cloning method it is favorable to use as Gram-negative bacterial strains, into which recombinant DNA is introduced, bacteria from *Escherichia coli* species, favourably strain TG1.

The method of optimizing the expression of synthetic genes of selected peptide fragments of natural myelin proteins, favorably is based on replacing the internal, constitutive promoter of the plasmid vector, replicating in *L. lactis* cells, with a regulated promoter, favorably with the promoter region of a gene deriving from the genome of *L. lactis* bacteria or other species or from the genome of a bacteriophage, preferably with the promoter region of *ptcB* gene engaged in sugar catabolism in lactic acid bacteria from *Lactococcus* genus, which is regulated by the presence in the medium of various sugars, such as: cellobiose, galactosa, salicin, esculin, glucose.

The method of optimizing the production of peptides in a bacterial system, favorably is based on culturing cells carrying the recombinant plasmid vector (with the cloned synthetic gene and a suitable promoter region, favorably the promoter of *ptcB* gene from the genome of *Lactococcus* bacteria) in a known medium specific for lactic acid bacteria, preferably on defined CDM medium (*chemically defined medium*), supplemented with sugar, favorably glucose or cellobiose, at a concentration of ≥ 0.5%, or on milk, whey or other suitable medium, at a temperature ranging between 20°-30°C, preferably at 30°C.

The nucleotide sequence of the promoter region of gene *ptcB,* comprising the -10 and -35 region, represented by formula 11 is also disclosed, for optimizing the production of synthetic gene encoding peptide fragment of natural myelin protein, according to the invention.

The method of producing the nucleotide sequence of the promoter region of *L. lactisptcB* gene as well as the method of replacing the internal, constitutive promoter of a plasmid vector that replicates in *L. lactis* with the promoter region of *L*. *lactis ptcB* gene, , is based on amplifying by PCR reaction the promoter region of *ptcB* gene using chromosomal DNA of *L. lactis* IL 1403 strain as template and pair of primers (ptcBfor and ptcBrev), which sequences are complementary to the DNA sequences flanking the promoter region of the chromosomal *ptcB* gene and modified in such a way that their 5' ends contain sequences recognized by specific restriction endonucleases, preferably Vspl (for primer ptcBfor) and Ncil (for primer ptcBrev) (Table 3). In result of the PCR reaction, by using such designed primers, a DNA fragment is obtained, which nucleotide sequence is represented by formula 12, comprising the sequence of the promoter region of *ptcB* gene (underlined).

The product obtained in this manner is subjected to digestion by restriction enzymes, preferably Vspl and Ncil, and then ligated with the recombinant vectors carrying synthetic gene encoding the selected peptide fragment of natural myelin protein, from which the original promoter region was removed (e.g. by digestion using the same restrictases). The recombinant DNA is introduced by electroporation method into cells of bacterial strains which are then cultured by known means. Cells containing the new promoter region are isolated from the cultured population, favorably by analyzing the obtained transformants by PCR technique for the presence of DNA fragments which expected length corresponds to the length of the selected promoter region of *ptcB* gene. Nucleotide sequences of the cloned DNA fragment are examined for conforminty with the nucleotide sequence of the promotor region of *ptcB* gene, by DNA sequencing technique.

The application of bacteria producing peptide fragment of natural myelin protein according to the invention, for induction of oral tolerance is also disclosed.

Application of the nucleotide sequence of the promoter region of *ptcB* gene, which comprises the -10 and -35 region, represented by formula **11**,, for optimizing the production of synthetic gene encoding peptide fragment of natural myelin protein according to the invention, for induction of oral tolerance.

It is expected that bacteria producing the peptide fragment of natural myelin protein, according to the invention, will induce oral tolerance as described in literature referring to oral administration of peptide mix in the form of the animal spinal cord hydrolysate, published in result of own studies (1, 6).

### Description of embodiments

### Materials and methods

### Bacterial strains, culture conditions and plasmid used.

Bacterial strains and plasmids used in the current studies are presented in Table 4. *Lactococcus lactis* strains were cultured in M17 medium (Oxoid, Anglia) supplemented with 0.5 % glucose or in defined CDM medium (*chemically defined medium*) supplemented with glucose or cellobiose (0.5% - 1 %) at a temperature between 20°C - 30°C. *Escherichia coli* strains were culture on Luria-Bertani (LB) medium, at a temperature of 37°C. When needed, for selection purposes, the following antibiotic were used: erythromycin 5 µg ml⁻¹ for *L. lactis* and ampicillin 100 µg ml⁻¹ for *E. coli.*

### Presented below are examples of implementing the invention

### Example

### Example I.

Synthetic genes synthesized by PCR method using for each peptide 2 complementary primers (for/rev"LONG") as a template and 2 short primers (for/rev"SHORT") homologous, respectively, to the extremities of the sequences of "LONG" primers (Table 1). In result of the PCR amplification reactions, products of expected length were obtained, which were then digested by enzymes Sall and Pstl and ligated with a plasmid replicating in *Lactococcus* bacterial cells - pIL253, digested beforehand with the same enzymes. Subsequently, the two DNA molecules were recombined with each other and introduced by electroporation method into the cells of selected *Lactococcus lactis strains,* which were cultured on solid M17 agar medium supplemented with 0.5% glucose and erythromycin at a final concentration of 5 µg ml⁻¹, and cells containing the expected synthetic genes were isolated from the cultured population by PCR method using specific primers. Nucleotide sequences of the cloned DNA fragments were examined for their conformity with the nucleotide sequences of the synthetic genes by DNA sequencing technique.

The resulting proper recombinant plasmids were isolated using a known method from *Lactococcus* bacterial cells and introduced by electroporation into cells of other lactic acid bacteria, e.g. *Bifidobacferium* and *Lactobacillus.*

Cell of *L. lactic* strains were transformed using a known electroporation technique (19). *Bifidobacterium* and *Lactobacillus* cells were transformed using a known electroporation technique, according to previously established procedures (20, 21).

Other techniques of molecular biology used in the example were in accordance with the standard methodology (22).

### Table 1

Sequence of primers ("LONG") used as templates for amplification of synthetic neuropeptide genes.

**Table 1**

| Name of neuropeptide | Name of primer | Primer sequence (forward/reverse) |
|---|---|---|
| MBP2 1-40 | FLON G_ MPB2 1 | 5' GAGTATTTCTATGGATCATGCTCGTCATG GTTTTTTACCACGTCATCGTGATACTGGT ATTTTAGATTCA 3' |
| | RLON G_ MPB2 1 | 5' TGAATCTAAAATACCAGTATCACGATGAC GTGGTAAAAAACCATGACGAGCATGATCC ATAGAAATACTC 3' |
| MOG3 5-55 | FLON G_ MOG3 5 | 5' GTATTTCTATGGAAGTTGGATGGTATCGT TCACCATTTTCACGTGTTGTTCATTTATAT CGTAATGG 3' |
| | RLON G_ MOG3 5 | 5' CCATTACGATATAAATGAACAACACGTGA AAATGGTGAACGATACCATCCAACTTCCT AGAAATAC 3' |
| MBP8 5-97 | FLON G_ MPB8 5 | 5' GTATTTCTATGCCAGGATCACGTCCACAT TTAATTCGTTTATTTTCACGT 3' |
| | RLON G_ MPB8 5 | 5' ACGTGAAAATAAACGAATTAAATGTGGAC GTGATCCTGGCATAGAAATAC 3' |
| PLP13 9-151 | FLON G_ PLP13 9 | 5' GTATTTCTATGCATTCATTAGGAAAATGGT TAGGACATCCAGATAAATTT 3' |
| | RLON G_ PLP13 9 | 5' AAATTTATCTGGATGTCCTAACCATTTTCC TAATGAATGCATAGAAATAC 3' |
| PLP17 8-191 | FLON G_ PLP17 8 | 5' GTATTTCTATGAATACATGGACAACATGTC AATCAATTGCTTTTCCATC 3' |
| | RLON G_ PLP17 8 | 5' GATGGAAAAGCAATTGATTGACATGTTGT CCATGTATTCATAGAAATAC 3' |

### Example II.

Production of selected peptides in lactic acid bacteria cells was based on expression of synthetic genes in bacterial cells by culturing them on defined CDM medium (*chemically defined medium*), supplemented with cellobiose at a concentration no less than 0.5%, at a temperature ranging between 20°-30°C, preferably at 30°C, until reaching optical density of OD 600³ 0.6.

### Example III.

Studies on expression of synthetic genes of selected myelin peptides on the transcriptional level were carried out using the RT-PCR method. Total RNA was isolated from bacterial strains carrying the recombinant plasmids and, after specific treatment, was subjected to reverse transcription reaction using reverse primers (revSHORT_peptide) complementary to the 3' end of the strand encoding the synthetic gene (Table 2). Obatined cDNA was subsequently subjected to amplification by the classical PCR technique using two primer pairs (forSHORTUniv and rev SHORT_peptide) (Table 2). In result of the experiment, two DNA fragments were obtained which length corresponded to the lenght of each of the genes.

### Table 2

Sequence of primers (,SHORT') used for amplification of synthetic neuropeptide genes.

**Table 2**

| Name of neuropep tide | Name of primer | Primer sequence |
|---|---|---|
| Universal for-ward primer | ForSHOR TUniv | 5' ACGCGTCGACAAGGAGTATTTCTA TG 3' |
| MBP21-4 0 | RevSHO RT_ MBP21 | 5' AACTGCAGTTATTATGAATCTAAAA TACCAG 3' |
| MOG35-55 | rev SHORT_ MOG35 | 5' AACTGCAGTTATTATTTTCCATTAC GATA 3' |
| MBP85-9 7 | rev SHORT_ MBP85 | 5' AACTGCAGTTATTAACGTGAAAATA AACG 3' |
| PLP139-151 | rev SHORT_ PLP139 | 5' AACTGCAGTTATTAAAATTTATCTG G 3' |
| PLP178-191 | rev SHORT_ PLP178 | 5'AACTGCAGTTATTATTTTGATGGA AAAGC 3' |

### Example IV.

Studies on expression of synthetic genes of selected myelin peptides on the translational level were carried out by immunoblot method, using protein extracts, obtained by known methods from cultures of bacterial cells carrying recombinant plasmids, suspendend in PBS buffer, pH 7.4 and adequate commercially available specific antibodies.

### Example V.

The promoter region of *ptcB* gene was amplified by PCR method using genomic DNA of *L. lactis* IL 1403 strain as template and specific primers (Table 3). In result of the PCR amplification, a product was obtained of expected length, which was subsequently digested with Ncil and Vspl enzymes and ligated with the pIL253 plasmid, replicating in *Lactococcus* bacteria cells, digested beforehand with the same enzymes. The recombinant DNA was introduced by electroporation into cells of *L. lactis* strain, which were cultured on solid M17 agar medium supplemented with 0.5% glucose and erythromycin at a final concentration of 5 µg ml⁻¹, and from the cultivated population cells containing the promoter region of *ptcB* gene were isolated using the PCR method and specific primers.

Cell of *L. lactic* strains were transformed using electroporation technique (19).

Other techniques of molecular biology used in the example were in accordance with the standard methodology (22).

### Table 3

Sequence of primers used for amplification of the promoter region of *ptcB* gene.

**Table 3**

| Name of primer | Primer sequence |
|---|---|
| ptcBfor | 5' GCGATTAATGTCGCCTAAAGGTTGC 3' |
| ptcBrev | 5' AATCCCGGCGTTTTATAATTTAACG TTC 3' |

### Example VI.

Synthetic peptide genes were amplified by PCR method using specific primers 'SHORT' (Table 2) and then ligated with a vector replicating in cells of *Escherichia coli* bacteria *-* pGEMT-Easy. Recombinant DNA was introdiced by electroporation method into cells of *E*. *coli* strain, which were cultivated on LB agar medium supplemented with ampicillin at a final concentration of 100 µg ml⁻¹, and from the cultivated population cells containing the expected synthetic genes were isolated using the PCR method and specific primers. Nucleotide sequences of the cloned DNA fragments were examined for conformity with nucleotide sequences of synthetic genes by DNA sequencing technique.

*E. coli* cells were transformed using electroporation technique (22).

Other molecular biology techniques used in the examples were in accordance with the standard methodology (22).

### Example VII.

The structure of the nucleotide sequence of synthetic genes of selected myelin peptides as well as of the promoter region of *ptcB* gene, according to the invention, was confirmed by sequencing of DNA fragments using the BigDye Terminator set (Promega, USA) and ABI377 sequencing apparatus (Applied Biosystem, USA), and recombinant plasmids as template as well as primers complementary to the extremities of each of the inserts. Obtained nucleotide sequences were analyzed using the BLAST program (23).

### Table 4

### Strains and plasmids.

**Table 4**

| Strain | genotype | source |
|---|---|---|
| *Lactococcus lactis* | | |
| IL1403 | Plasmid-free laboratory strain | (24) |
| IBB360 | Strain with potential autolytic properties | J. Bardowski -IBB PAN collection |
| IBB477 | Strain with potential adhesive properties, Tc^{r} | J. Zycka-Krzesinska -IBB PAN collection |

| *Bifidobacterium* | | |
|---|---|---|
| *Bifidobacterium pseudocatenulat um* M115 | Human intestinal isolate; plasmid-free | IPLA Laboratory Collection |

| *Lactobacillus* | | |
|---|---|---|
| *Lactobacillus plantarum* | NCFB1193 | NCFB Collection |

| *Escherichia coli* | | |
|---|---|---|
| TG1 | supE Q(hsdM-mcrB)(rk-mk-McrB -)thi Q(lac-proAB)F'[traD36 laclq lacZ QM15 proA+B+] | (25) |

| Plazmid | | |
|---|---|---|
| pIL253 | Ery^{r}, vector for cloning genes in *Lactococcus* bacteria | (26) |
| pIL253_p[*ptcB*] | Ery^{r}, vector for cloning genes in *Lactococcus* bacteria, with regulated promoter region of *ptcB* gene | This work |
| pGEMT-Easy | Amp^{R}, vector for cloning genes in *Escherichia* bacteria | Promega |

| | | |
|---|---|---|
| * Ery^{r} - erythromycin resistance Amp^{r} - ampicillin resistance | | |

### Sequence listing free text

### MBP21-40:

ATGGATCATGCTCGTCATGGTTTTTACCACGTCATCGTGATACTGGT
ATTTTAGATTCA

**formula** 1
MDHARHGFLPRHRDTGILDS
**formula 1a**

### MOG35-55:

ATGGAAGTTGGATGGTATCGTTCACCATTTTCACGTGTTGTTCATTTA
TATCGTAATGGAAAA

**formula 2**
MEVGWYRSPFSRVVHLYRNGK
**formula 2a**

### MBP85-97:

CCAGGATCACGTCCACATTTAATTCGTTTATTTTCACGT
**formula 3**

PGSRPHLIRLFSR
**formula 3a**

### PLP139-151:

CATTCATTAGGAAAATGGTTAGGACATCCAGATAAATTT
**formula 4**

HSLGKWLGHPDKF
**formula 4a**
PLP178-191:
ATGAATACATGGACAACATGTCAATCAATTGCTTTTCCATCAAAA
**formula 5**
NTWTTCQSIAFPSK
**formula 5a**

### DNA fragment containing synthetic gene of MBP 21-40 peptide

5'ACGCGTCGACAAGGAGTATTTCTATGGATCATGCTCGTCATGGTTTT
TTACCACGTCATCGTGATACTGGTATTTTAGATTCATAATAACTGCAGT
T3'

**formula 6**

### DNA fragment containing synthetic gene of MOG35-55 peptide

5'ACGCGTCGACAAGGAGTATTTCTATGGAAGTTGGATGGTATCGTTC
ACCATTTTCACGTGTTGTTCATTTATATCGTAATGGAAAATAATAACTG
CAGTT3'

**formula 7**

### DNA fragment containing synthetic gene of MBP85-97 peptide

5'ACGCGTCGACAAGGAGTATTTCTATGCCAGGATCACGTCCACATTT

AATTCGTTTATTTTCACGTTAATAACTGCAGTT3'

**formula 8**

### DNA fragment containing synthetic gene of PLP139-151 peptide

5'ACGCGTCGACAAGGAGTATTTCTATGCATTCATTAGGAAAATGGTTA
GGACATCCAGATAAATTTTAATAACTGCAGTT3'

**formula 9**

### DNA fragment containing synthetic gene of PLP178-191 peptide

5'ACGCGTCGACAAGGAGTATTTCTATGAATACATGGACAACATGTCA
ATCAATTGCTTTTCCATCAAAATAATAACTGCAGTT3'

**formula 10**

### promoter region of the ptcB gene

GTCGCCTAAAGGTTGCTATAAAGCGAAAAATAAACTAATTAACAAATT
ATATAAAGCACTTTCAAAAAATAGATAACGCTTGCATTATGAAAATGAA
AACGTTATAATTATTTTTATAAAGAACGTTAAATTATAAAACG

**formula 11**

### DNA fragment containing the promoter region of the ptcB gene

GCGATTAATGTCGCCTAAAGGTTGCTATAAAGCGAAAAATAAACTAAT
TAACAAATTATATAAAGCACTTTCAAAAAATAGATAACGCTTGCATTAT
GAAAATGAAAACGTTATAATTATTTTTATAAAGAACGTTAAATTATAAAA
CGCCGGGATT

**formula 12**

### MBP

MGNHAGKRELNAEKASTNSETNRGESEKKRNLGELSRTTSEDNEVFGE
ADANQNNGTSSQDTAVTDSKRTADPKNAWQDAHPADPGSRPHLIRLFS
RDAPGREDNTFKDRPSESDELQTIQEDSAATSESLDVMASQKRPSQRH
GSKYLATASTMDHARHGFLPRHRDTGILDSIGRFFGGDRGAPKRGSGK
DSHHPARTAHYGSLPQKSHGRTQDENPVVHFFKNIVTPRTPPPSQGKG
RGLSLSRFSWGAEGQRPGFGYGGRASDYKSAHKGFKGVDAQGTLSKIF
KLGGRDSRSGSPMARR

**formula 13**

### PLP

GLLECCARCLVGAPFASLVATGLCFFGVALFCGCGHEALTGTEKLIETYF
SKNYQDYEYLINVIHAFQYVIYGTASFFFLYGALLLAEGFYTTGAVRQIFG

DYKTTICGKGLSATVTGGQKGRGSRGQHQAHSLERVCHCLGKWLGHP
DKFVGITYALTVVWLLVFACSAVPVYIYFNTWTTCQSIAFPSKTSASIGSL
CADARMYGVLPWNAFPGKVCGSNLLSICKTAEFQMTFHLFIAAFVGAAA
TLVSLLTFMIAATYNFAVLKLMGRGTKF

**formula 14**

### MOG

MASLSRPSLPSCLCSFLLLLLLQVSSSYAGQFRVIGPRHPIRALVGDEVEL
PCRISPGKNATGMEVGWYRPPFSRVVHLYRNGKDQDGDQAPEYRGRT
ELLKDAIGEGKVTLRIRNVRFSDEGGFTCFFRDHSYQEEAAMELKVEDPF
YWVSPGVLVLLAVLPVLLLQITVGLVFLCLQYRLRGKLRAEIENLHRTFDP
HFLRVPCWKITLFVIVPVLGPLVALIICYNWLHRRLAGQFLEELRNPF

**formula 15**

### Citation list

1. Kwiatkowska-Patzer B, Michalkiewicz J, Zielinska J, Kasarello K, Kurzepa K and Lipkowski A.W. 2009. Pig spinal cord hydrolyzate ameliorates immunological reaction in experimental allergic encephalomyelitis. Acta Neurobiol Exp. 69: 73-78.
2. Moingeon P, van Overtvelt L and Razafindratsita A. Miedzynarodowy patent nr. WO2006/123230. Compositions for antigen-specific induction of tolerance.
3. Carnegie PR. 1971. Amino acid sequence of the encephalitogenic basic protein from human myelin. Biochem. J. 123: 57-67.
4. Stoffel W, Giersiefen H, Hillen H, Schroeder W and Tunggal B. 1985. Amino-acid sequence of human and bovine brain myelin proteolipid protein (lipophilin) is completely conserved. Biol. Chem. Hoppe-Seyler 366: 627-635.
5. Hilton AA, Slavin AJ, Hilton DJ and Bernard CCA. 1995. Characterization of cDNA and genomic clones encoding human myelin oligodendrocyte glycoprotein. J. Neurochem. 65: 309-318.
6. Kwiatkowska-Patzer B, Baranowska B, Barcikowska-Litwin M and Lipkowski AW. Suppression of experimental autoimmune encephalomyelitis in the rat by oral administration of spinal cord protein hydrolysate, in: Neurochemistry (Teelken and Korf, eds), Plenum Press, N. York 1997, pp. 137-140.
7. Libudzisz Z, Walczak P and Bardowski J. (eds.) - Lactic acid bacteria - classification, metabolism, genetics, applications (in polish), monograph, Edition of Technical University of odz, odz 1998, 2003 and 2004.
8. Teusink B and Smid EJ. Modelling strategies for the industrial exploitation of lactic acid bacteria. 2006. Nat Rev Microbiol. 4: 46-56.
9. Lin DC. 2003. Probiotics as functional foods. Nutr Clin Pract. 18:497-506.
10. Ouwehand AC. Salminen S and Isolauri E. 2002. Probiotics: an overview of beneficial effects. Antonie Van Leeuwenhoek. 82: 279-89.
11. Bermudez-Humaran LG. Cortes-Perez NG, Lefevre F, Guimaraes V. Rabot S, Alcocer-Gonzalez JM, Gratadoux JJ, Rodriguez-Padilla C. Tamez-Guerra RS. Corthier G. Gruss A and Langella PA. 2005. A novel mucosal vaccine based on live lactococci expressing E7 antigen and IL-12 induces systemic and mucosal immune responses and protects mice against human papillomavirus type 16-induced tumors. J Immunol. 175: 7297-302.
12. Vaughan EE. de Vries MC. Zoetendal EG. Ben-Amor K, Akkermans AD and de Vos WM. 2002. The intestinal LABs. Antonie Van Leeuwenhoek. 82: 341-52.
13. Barcellos LF, Oksenberg JR, Begovich AB, Martin ER, Schmidt S, Vittinghoff E, Goodin DS, Pelletier D, Lincoln RR, Bucher P, Swerdlin A, Pericak-Vance MA, Haines JL and Hauser SL. Multiple Sclerosis Genetics Group. 2003. HLA-DR2 dose effect on susceptibility to multiple sclerosis and influence on disease course. Am. J. Human Gen. 72: 710-716.
14. Steinman L and Zamvil S. 2003. Transcriptional analysis of targets in multiple sclerosis. Nature Rev. Immun. 3: 483-492.
15. Pedotti R, DeVoss JJ, Youssef S, Mitchell D, Wedemeyer J, Madanat R, Garren H, Fontoura P, Tsai M, Galli SJ, Sobel RA and Steinman L. 2003. Multiple elements of the allergic arm of the immune response modulate autoimmune demyelination. Proc. Natl. Acad. Sci. USA. 100: 1867-1872.
16. Anderson AC, Nicholson LB, Legge KL, Turchin V, Zaghnanni H and Kuchroo K. 2000. High frequency of autoreactive myelin proteolipid protein specific T cell in the perophery of naive mice: mechanism of selection of the self-reactive repertoire. J Exp Med. 6: 56-61.
17. Costa O, Divoux D, Ischenko A, Tron F, Fontaine M. 2003. Optimisation of an animal model of experimental autoimmune encephalomyelitis achieved with a multiple MOG(35-55) peptide in C57BL6/J strain of mice. Journal of Autoimmunity. 20: 51-61.
18. Zamvil SS, Nelson PA, Mitchell DJ, Knobler RL, Fritz RB and Steinman L. 1985. Encephalitogenic Tcell clones specific for myelin basic protein .An unusual bias in antigen recongnition. J Exp Med. 162: 2107-2124.
19. Holo H and Nes IF. 1989. High-frequency transformation, by electroporation, of Lactococcus lactis subsp. cremoris with glycine in osmotically stabilized media. Appl. Environ. Microbiol. 55: 3119.
20. Álvarez-Martín P, Flórez AB, Margolles A, del Solar G and Mayo B. 2008. Improved cloning vectors for Bifidobacteria, based on the Bifidobacterium catenulatum pBC1 replicon. 74: 4656-4665.
21. Aukrust TW, Brurberg MB and Nes IF. 1995. Transformation of Lactobacillus by electroporation. Methods Mol Biol. 47: 201-208.
22. Sambrook J., Fritsche EF and Maniatis T. 2001. Molecular cloning: a laboratory manual. Cold Spring Harbor Laboratory, Cold Spring Harbor, N.Y.
23. Altschul SF, Gis W, Miller W, Myers EW and Lipman DJ. 1990. Basic local alignment search tool. J. Mol. Biol. 215: 403.
24. Chopin A, Chopin MC, Moillo-Batt A and Langella P. 1984. Two plasmid-determined restriction and modification systems in Streptococcus lactis. Plasmid 11, 260-263.
25. Gibson TJ. 1984. Studies on the Eppstein-Barr virus genome. Ph.D. Thesis, Cambridge University, Cambridge, England.

Simon D and Chopin A. 1988. Construction of a vector plasmid family for molecular cloning in Streptococcus lactis Biochimie. 70: 559-566

## Claims

1. Synthetic gene for use in the induction of oral tolerance in the treatment of multiple sclerosis, wherein the gene contains the MBP21-40 nucleotide fragment ATGGATCATGCTCGTCATGGTTTTTTACCACGTCATCGTGATACTGGTATTTTAGATTCA (formula 1), encoding a peptide consisting of amino acid sequence MDHARHGFLPRHRDTGILDS (formula 1a), said peptide being a fragment of a natural myelin protein.

## Patentansprüche

1. Synthetisches Gen zur Verwendung in der Induktion von oraler Toleranz bei der Behandlung von Multipler Sklerose, wobei das Gen das MBP21-40 Nukleotidfragment ATGGATCATGCTCGTCATGGTTTTTTACCACGTCATCGTGAT ACTGGTATTTTAGATTCA (Formel 1) enthält, das für ein Peptid kodiert, das aus der Aminosäuresequenz MDHARHGFLPRHRDTGILDS (Formel la) besteht, wobei das Peptid ein Fragment eines natürlichen Myelinproteins ist.

## Revendications

1. Gène synthétique pour utilisation dans l'induction d'une tolérance orale dans le traitement de la sclérose en plaques, dans lequel le gène contient le fragment nucléotidique MBP21-40 ATGGATCATGCTCGTCATGGTTTTTTACCACGTCATCGTGATACTGGTATTTTAGATTCA (formule 1), codant pour un peptide consistant en la séquence d'acides aminés MDHARHGFLPRHRDTGILDS (formule 1a), ledit peptide étant un fragment d'une protéine naturelle de la myéline.
